# FASCICULE DE BREVET EUROPEEN

(11) **EP 2 508 227 B1**
(45) Date de publication et mention de la délivrance du brevet: **25.12.2013**
(21) Numéro de dépôt: 12153644.5
(22) Date de dépôt: 02.02.2012
(51) Int. Cl.: A61N 1/365, A61N 1/362, A61N 1/368

(54) **Prothèse cardiaque implantable de resynchronisation par stimulation biventriculaire, comprenant des moyens de remodelage inverse**
Implantierbare Herzprothese zur Resynchronisation des Herzens durch biventrikuläre Stimulation, die Mittel zur inversen Herzrekonfiguration umfasst
Implantable cardiac prosthesis for resynchronisation by bi-ventricular stimulation, including a reverse remodelling means

(30) Priorité: 06.04.2011 FR 1152971
(43) Date de publication de la demande: 10.10.2012
(73) Titulaire: Sorin CRM SAS, 92140 Clamart (FR)
(72) Inventeur: Heurteau, Mélanie, 92160 Antony (FR); Casset, Cyrille, 33650 Saint Selve (FR)
(74) Mandataire: Dupuis-Latour, Dominique

(56) Documents cités:
- EP-A1- 2 070 562
- EP-A2- 1 867 360
- US-A1- 2002 161 410
- US-A1- 2007 179 542

## Description

L'invention concerne les "dispositifs médicaux implantables actifs" tels que définis par la directive 90/385/CEE du 20 juin 1990 du Conseil des communautés européennes.

Elle concerne plus précisément les implants permettant de surveiller en continu le rythme cardiaque et délivrer si nécessaire au coeur des impulsions électriques permettant d'assurer une stimulation conjointe et permanente des ventricules gauche et droit afin de resynchroniser ces derniers, technique dite "CRT" (*Cardiac Resynchronization Therapy*) ou "BVP" *(Bi-Ventricular Pacing).*

Un tel stimulateur CRT est par exemple divulgué dans le EP 1 108 446 A1 (ELA Medical).

Le dispositif applique entre les instants respectifs de stimulation des ventricules gauche et droit un délai dit "délai interventriculaire" (DVV ou VVD) ajusté de manière à resynchroniser la contraction des deux ventricules pour optimiser l'état hémodynamique du patient.

En effet, une stimulation simultanée des deux ventricules n'est pas toujours optimale, car elle n'aboutit pas forcément à une contraction synchrone des deux ventricules du fait, d'une part, des délais de conduction au sein du myocarde qui ne sont pas les mêmes à droite et à gauche et peuvent dépendre de multiples facteurs et, d'autre part, de l'emplacement de la sonde ventriculaire gauche, selon que celle-ci est une sonde enfilée dans le sinus coronaire ou une sonde épicardique. Il est donc souhaitable d'établir un délai entre les deux stimulations, et d'ajuster ce délai pour resynchroniser la contraction des ventricules et assurer ainsi une optimisation fine de l'hémodynamique. Le DVV pourra être nul, positif (le ventricule gauche étant stimulé après le ventricule droit), ou négatif (le ventricule droit étant stimulé après le ventricule gauche).

On notera que la stimulation d'un ventricule donnée (droit ou gauche) peut être réalisée en un site de stimulation unique, ou simultanément en une pluralité de sites.

On appellera "sites de stimulation" l'emplacement physique des électrodes intracardiaques par rapport au tissu du myocarde. Ces sites sont choisis au moment de l'implantation, par un positionnement approprié des électrodes après vérification de l'efficacité des sites choisis. Dans certains cas, le dispositif multisite comporte plusieurs électrodes placées dans une même cavité, et un changement de site de stimulation dans cette cavité est alors possible par une commutation interne du dispositif.

Les dispositifs CRT incluent en outre un mode de fonctionnement "double chambre" classique dans lequel le dispositif surveille l'activité ventriculaire après un événement auriculaire spontané (détection d'une onde P de dépolarisation auriculaire) ou stimulé (application d'une impulsion A de stimulation auriculaire). En même temps, le dispositif commence à compter un délai dit "délai atrioventriculaire" (DAV ou AVD) tel que si aucune activité spontanée ventriculaire (onde R) n'a été détectée à l'issue de ce délai, alors le dispositif déclenche une stimulation de ce ventricule (application d'une impulsion V).

On désignera par la suite de façon globale par "configuration de stimulation" la combinaison des caractéristiques i) relatives aux "sites de stimulation" (position physique et/ou sélection des sites parmi plusieurs possibles) et de celles ii) relatives au paramétrage des délais DVV et DAV.

Le EP 1 736 203 A1 (ELA Medical) décrit une technique permettant d'évaluer de façon simple, rapide, automatisée et précise l'incidence des différents paramètres de la thérapie CRT, notamment des délais DAV et DVV et du choix des sites de stimulation, de manière à optimiser l'état hémodynamique du patient.

Le dispositif décrit par ce document utilise à cet effet les paramètres liés à l'accélération endocardiaque (EA) pour déterminer la configuration de stimulation optimale, au moment de l'implantation ou ultérieurement. L'accélération endocardiaque est par exemple mesurée par un accéléromètre intégré dans une sonde endocavitaire, comme décrit par exemple dans le EP 0 515 319 A1 (Sorin Biomedica Cardio SpA)

En effet, diverses études cliniques ont montré que l'accélération endocardiaque est un paramètre qui reflète très précisément et en temps réel les phénomènes liés aux contractions et relaxations de la cavité cardiaque, et permet ainsi de fournir des informations très complètes sur la mécanique cardiaque, aussi bien dans le cas d'un fonctionnement normal que d'un fonctionnement déficient.

Pour optimiser automatiquement la configuration de stimulation, dans un mode de test déclenché périodiquement par l'implant le EP 1 736 203 A1 propose de modifier la configuration courante et de déterminer pour chaque configuration testée un indice de performance dérivé d'un ou plusieurs paramètres relatifs au pic d'accélération endocardiaque PEA, et reflétant l'efficacité de la configuration choisie. La configuration finalement choisie sera celle qui maximise cet indice de performance.

Un technique comparable, utilisant une combinaison de plusieurs indices spécifiques, est décrite dans le EP 2 070 562 A1 (ELA Medical).

Toutes les techniques décrites par ces documents ont pour objet de déterminer une configuration optimale de stimulation, c'est-à-dire une configuration qui, à un moment donné de la thérapie, optimise les paramètres hémodynamiques du patient, en particulier pour augmenter la contractilité du myocarde et améliorer le remplissage des cavités et par voie de conséquence le débit cardiaque.

La présente invention, à la différence de ces techniques connues, n'a en revanche pas pour objet de déterminer une configuration optimale de stimulation.

L'invention se rapporte à un phénomène dénommé "remodelage cardiaque", qui peut être défini comme l'ensemble des modifications du coeur en réponse à une pathologie et qui est généralement associé à un plus mauvais pronostic.

Ce remodelage se manifeste à la longue par une augmentation de la taille du ventricule gauche, avec dégradation de la fraction d'éjection et du régime des pressions intraventriculaires du fait de la baisse de contractilité et/ou de pressions trop élevées en aval, et *in fine* une diminution du débit cardiaque entraînant de graves conséquences sur l'organisme par progression de l'insuffisance cardiaque.

En stimulant de façon contrôlée les ventricules en au moins deux points, la thérapie CRT permet d'optimiser le cycle contraction/relaxation, avec un bénéfice direct en facilitant le travail du coeur, mais sans pour autant qu'il y ait régression des modifications antérieures survenues du fait du remodelage. De plus, chez certains patients ont ne constate pas de réponse significative à la stimulation CRT, qui n'apporte donc aucun bénéfice.

Pour inverser les effets du remodelage, il a été proposé des moyens médicamenteux, notamment le traitement par bêtabloquants, ainsi que des moyens non médicamenteux incluant certaines techniques chirurgicales de reconstruction du ventricule gauche, ou encore l'utilisation de certains dispositifs médicaux de contention mécanique passive ou d'assistance mécanique cardiaque.

Le problème de l'invention est de trouver un mode de fonctionnement du dispositif CRT qui permette de pallier efficacement les conséquences délétères du remodelage cardiaque.

Essentiellement, l'idée de base de l'invention consiste à modifier de façon très temporaire la configuration de stimulation dans un sens allant à l'encontre d'une optimisation des paramètres hémodynamiques.

Le but de cette manoeuvre est de provoquer une modification immédiate de la réponse hémodynamique du myocarde à la stimulation contrôlée et de forcer le coeur à "réagir" pour s'adapter à ce mode de stimulation non-optimal avec pour conséquence une amélioration de la contractilité et, sur le long terme, de la morphologie du coeur. En d'autre termes, la stimulation spécifique produit un remodelage inverse par modification lente (sur plusieurs mois) de la réponse physiologique du coeur sous cette stimulation contrainte.

Selon *un premier aspect de l'invention,* l'analyse de la réponse transitoire du coeur au changement de configuration permet d'évaluer la plus ou moins grande réactivité du myocarde à tel ou tel brusque changement (délibéré) de configuration, de manière à rechercher parmi plusieurs configurations celle qui - si elle était appliquée de façon permanente - serait potentielle la plus délétère.

Plus particulièrement, l'invention utilise à cet effet un capteur hémodynamique tel qu'un capteur d'accélération endocardiaque. Un tel capteur est en lui-même connu, mais les informations qu'il délivre ne sont pas utilisées pour optimiser les cycles cardiaques à venir - comme dans les techniques proposées par les dispositifs antérieurs cités plus haut -, mais pour sélectionner parmi divers modes de stimulation appliqués celui (ou ceux) qui permettra d'optimiser le remodelage inverse du muscle cardiaque.

Selon *un deuxième aspect de l'invention,* l'analyse ainsi réalisée permettra de définir des séquences temporaires - ponctuellement délétères - de stimulation, applicables au myocarde afin de forcer celui-ci à effectuer une adaptation forte permettant, sur le long terme, d'améliorer sa réponse en termes de pression, de volume éjecté et de délais de remplissage et d'éjection.

Ces séquences de stimulation spécifique contrôlée, qui ne dureront que quelques secondes ou minutes, n'auront pas d'effet délétère sur le long terme, mais au contraire forceront le coeur à réagir dans le sens d'un remodelage inverse, dont les conséquences bénéfiques pourront être constatées au bout de quelques jours, quelques semaines ou quelques mois. Il sera notamment possible de réaliser des programmes d"'entraînement" (par exemple quotidiens), composés de plusieurs séquences successives de ce type, avec retour final à la configuration d'origine. Les séquences d'application d'une configuration modifiée pourront être éventuellement entrecoupées de périodes de récupération, avec stimulation à la configuration d'origine, avant application d'une autre séquence spécifique.

Plus précisément, l'invention propose un dispositif médical implantable actif de type prothèse cardiaque de resynchronisation par stimulation biventriculaire, comprenant : des moyens de détection d'événements auriculaires et ventriculaires ; des moyens aptes à appliquer des impulsions à des sites de stimulation des ventricules droit et gauche ; des moyens de calcul d'un délai atrioventriculaire, DAV ; et des moyens de calcul d'un délai interventriculaire, DVV. La combinaison des sites de stimulation, du DAV et du DVV définissant ensemble une configuration de stimulation. Le dispositif comprend également : des moyens de recueil d'un signal d'accélération endocardiaque ; des moyens de mesure de l'amplitude crête-à-crête du premier pic d'accélération endocardiaque, PEA, à chaque cycle cardiaque ; et des moyens de commutation, aptes à changer de façon contrôlée et temporaire la configuration courante, d'une configuration originelle à une configuration modifiée.

De façon caractéristique de l'invention, ce dispositif comprend en outre des moyens aptes à évaluer, dans la configuration modifiée, un ensemble de données comprenant : la valeur maximale d'amplitude du PEA atteinte sur une première période temporelle après le changement de configuration ; la valeur moyenne du PEA, calculée sur un nombre prédéterminé de cycles cardiaques commençant après une deuxième période temporelle, de stabilisation, après le changement de configuration ; la variabilité du PEA autour de ladite valeur moyenne de PEA ; et la durée d'une troisième période temporelle, de stabilisation, après le changement de configuration.

Selon diverses caractéristiques subsidiaires avantageuses :
- le dispositif comprend des moyens de séquencement, pour appliquer des séquences de stimulation selon des configurations différentes, avec alternance, pendant des périodes temporelles prédéterminées, entre ladite configuration originelle et une configuration modifiée choisie parmi une pluralité de configurations modifiées différentes possibles, la configuration modifiée étant changée à chaque alternance ;
- ces moyens de séquencement sont aptes, après chaque application d'une configuration modifiée et retour à la configuration originelle, à maintenir cette configuration originelle pendant une durée de récupération prédéterminée ;
- le dispositif comprend des moyens aptes à constituer une table à deux entrées donnant ledit ensemble de données évalué pour chaque couple {configuration originelle, configuration modifiée} successivement appliqué ;
- le dispositif comprend des moyens pour désigner une configuration représentative de la réaction maximale du myocarde au changement de configuration, notamment celle minimisant la valeur moyenne du PEA par rapport à la valeur maximale d'amplitude du PEA ;
- la durée de la troisième période temporelle, de stabilisation, après le changement de configuration est évaluée par recherche de la durée nécessaire pour qu'une moyenne à court terme du PEA soit égale à une moyenne à long terme du PEA, à un écart autorisé prédéterminé près ;
- la variabilité du PEA autour de ladite valeur moyenne de PEA est évaluée par calcul d'un écart-type du PEA sur un nombre prédéterminé de cycles consécutifs.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre les différents signaux caractérisant l'activité du coeur au cours d'un cycle cardiaque, avec un relevé d'électrocardiogramme de surface ECG et les variations correspondantes du signal d'accélération endocardiaque EA.
La Figure 2 est un chronogramme montrant les variations de l'accélération endocavitaire EA au cours de trois cycles cardiaques successifs.
La Figure 3 est un chronogramme illustrant les variations de l'amplitude de crête du pic d'accélération endocardiaque au cours de cycles successifs, avant et après un changement brusque de configuration de stimulation.

On va maintenant décrire un exemple de réalisation de l'invention, en référence aux dessins annexés.

L'invention peut notamment être appliquée aux dispositifs implantables tels que ceux de la famille *Paradym CRT* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre certaines des fonctions de l'invention qui seront décrites ci-dessous (optimisation des paramètres et suivi de l'état du patient). L'adaptation de ces appareils à la mise en oeuvre des fonctions de l'invention est à la portée de l'homme du métier, et elle ne sera pas décrite en détail.

La technique de l'invention est basée sur l'analyse de l'accélération endocardiaque (ci-après désignée "EA"), qui est un paramètre qui reflète très précisément et en temps réel les phénomènes concourant au fonctionnement mécanique du myocarde et qui peut être mesuré par un accéléromètre couplé au muscle cardiaque, comme cela est décrit par exemple dans le EP 0 515 319 A1 (Sorin Biomedica Cardio SpA). Ce document enseigne la manière de recueillir un signal EA au moyen d'une sonde endocavitaire pourvue d'une électrode distale de stimulation implantée dans l'oreillette ou le ventricule et intégrant un microaccéléromètre permettant de mesurer l'accélération endocardiaque.

On notera toutefois que, bien que dans la présente description on fasse principalement référence à l'analyse d'un signal EA délivré par un capteur placé sur une sonde endocavitaire, l'invention est également applicable à une analyse opérée à partir d'un signal EA délivré par d'autres types de capteurs implantés, tels qu'un capteur de mouvement d'une paroi du myocarde, un capteur épicardique ou un accéléromètre placé dans le boîtier d'un implant. L'invention est également applicable à l'analyse d'un signal EA externe recueilli de manière non invasive, par exemple issu d'un capteur fixé sur la poitrine du patient au niveau du sternum.

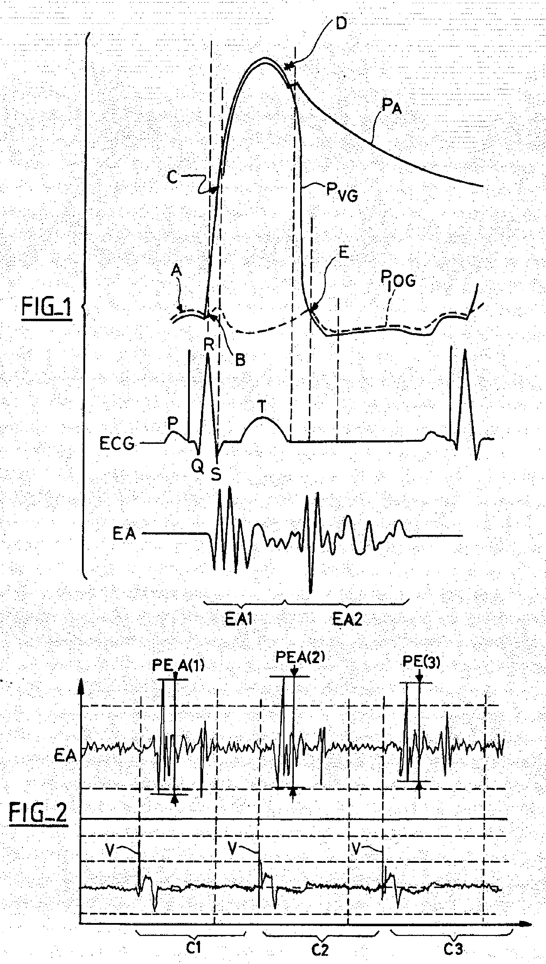

La Figure 1 illustre les différents signaux caractérisant l'activité du coeur au cours d'un cycle cardiaque, avec : le profil des pressions intracardiaques (P_{A}, P_{VG} et P_{OG}), un relevé d'électrocardiogramme de surface (ECG), et les variations du signal d'accélération endocardiaque (EA).

La caractéristique P_{A} illustre les variations de la pression aortique, P_{VG} celles du ventricule gauche et P_{OG} celles dans l'oreillette gauche. Les points A à E correspondent aux différentes phases suivantes : A) contraction de l'oreillette gauche, B) fermeture de la valvule mitrale, C) ouverture de la valvule aortique, D) fermeture de la valvule aortique, et E) ouverture de la valvule mitrale.

Le signal ECG présente successivement : l'onde P correspondant à la dépolarisation des oreillettes, le complexe QRS correspondant à la dépolarisation des ventricules et l'onde T de repolarisation ventriculaire.

Le signal d'accélération endocardiaque EA recueilli au cours d'un cycle cardiaque donné (ci-après "signal EA") forme deux composantes principales, correspondant aux deux bruits majeurs du coeur (sons S1 et S2 du phonocardiogramme) qu'il est possible de reconnaître dans chaque cycle cardiaque :
- la composante EA1, débutant à la suite du complexe QRS, est engendrée par une combinaison de la fermeture des valves auriculo-ventriculaires, de l'ouverture des valves semi-lunaires et de la contraction du ventricule gauche. Les variations d'amplitude de cette composante EA1 sont étroitement liées aux variations de la pression dans le ventricule (l'amplitude maximale crête-à-crête étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ; et
- la composante EA2 survient pendant la phase de relaxation ventriculaire isovolumique. Elle accompagne la fin de la systole ventriculaire et est principalement produite par la fermeture des valves aortiques et pulmonaires.

La Figure 2 illustre les variations du signal EA sur trois cycles successifs C1, C2, C3.

Des marqueurs de début de cycle permettent d'individualiser les cycles cardiaques successifs dans le signal EA recueilli en continu, et d'isoler des sous-signaux EA bornés dans le temps et correspondant chacun à une durée d'un seul cycle cardiaque. Dans le cas d'un signal EA endocavitaire, ces marqueurs temporels de début de cycle peuvent être fournis par l'implant lui-même, qui, selon le mode de fonctionnement, garde en mémoire les instants de la stimulation V (visible sur le tracé d'ECG du bas sur la Figure 2), ou bien les instants de détection de l'onde R, en mode non stimulé.

Le dispositif mesure alors l'amplitude crête-à-crête, notée PEA(i), du pic d'accélération endocardiaque EA (ci-après "PEA") sur le cycle i.

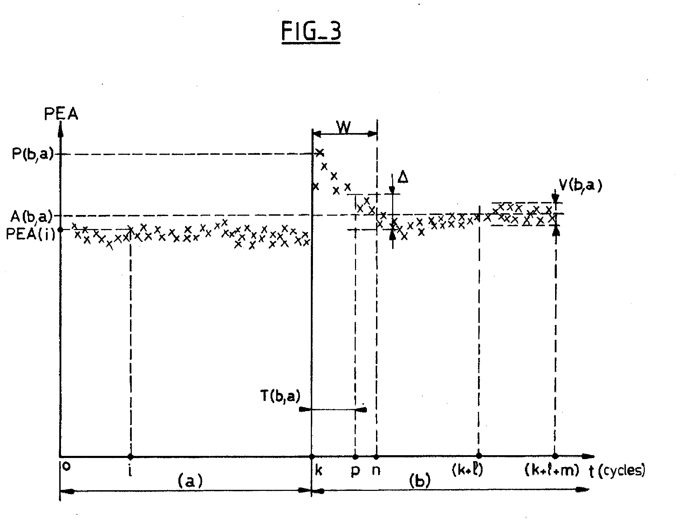

La Figure 3 illustre l'évolution de cette mesure PEA(i) au cours du temps, cycle après cycle.

Plus précisément, on a représenté sur cette figure les variations de PEA(i) lors d'un changement de configuration de stimulation, depuis une configuration d'origine, notée a, vers une configuration modifiée spécifique, notée b, le changement de configuration étant opéré au cycle k.

Comme indiqué en introduction, on entendra par "configuration de stimulation" la combinaison des caractéristiques i) relatives aux "sites de stimulation" (position physique et/ou sélection des sites parmi plusieurs possibles) et de celles ii) relatives au paramétrage des délais DVV et DAV.

Le changement brusque de configuration de stimulation au cycle k va entraîner une réaction physiologique du myocarde, par exemple, comme illustré Figure 3, une augmentation de l'amplitude du PEA puis une stabilisation progressive de cette amplitude à une valeur moyenne un peu plus élevée (dans l'exemple illustré) que dans la configuration a précédente.

Le dispositif évalue les données suivantes, consécutives à la transition brusque de la configuration a à la configuration b :
- P(b,a) : la valeur maximale du PEA atteinte sur une période temporelle W après le changement de configuration. Cette valeur maximale est recherchée dans la fenêtre W comprise entre les cycles k (changement de configuration) et n, (n-k) définissant la largeur de la fenêtre W, typiquement (n-k) = 10 cycles ;
- A(b,a) : la valeur moyenne du PEA, calculée sur un nombre prédéterminé de cycles cardiaques commençant après une période de stabilisation suivant le changement de configuration. Cette moyenne est évaluée sur un nombre prédéterminé m de cycles après que l'amplitude du PEA se soit stabilisée, typiquement m = 10 cycles ;
- T(b,a) : la durée de stabilisation après le changement de configuration. Le critère de stabilisation peut être par exemple défini comme le temps compris entre le moment (instant k) de changement de configuration de a vers b jusqu'au moment où une moyenne à court terme (par exemple sur 4 cycles consécutifs) du PEA est égale, à un pourcentage prédéterminé près, à une moyenne à long terme (par exemple sur 30 cycles consécutifs du PEA : si ce critère est vérifié au cycle p, la durée de stabilisation T(b,a) sera ainsi définie comme la durée (p-k), exprimée en nombre de cycles ; et
- V(b,a) : la variabilité du PEA autour de la valeur moyenne A(b,a) après stabilisation. Cette donnée est par exemple obtenue par un calcul d'écart-type effectué sur m cycles consécutifs entre les cycles (k+l) et (k+l+m), étant supposé qu'après le cycle (k+l) l'amplitude du PEA pourra dans tous les cas être considérée comme stabilisée autour de sa valeur moyenne A(b,a).

On obtient ainsi un quadruplet de données {P(b,a), A(b,a), T(b,a), V(b,a)}, représentatif de la transition de la configuration a à la configuration b. Ces mesures sont réitérées pour une ou plusieurs autres configurations différentes, de manière automatique ou manuelle, et un tableau correspondant est dressé, donnant pour chaque transition d'un mode d'origine (en colonne) à un mode modifié (en ligne), les quadruplets correspondants {P, A, T, V} :

**Tableau 1**

| | Configuration #1 | Configuration #2 | Configuration #3 |
|---|---|---|---|
| Configuration #1 | - | {P, A, T, V} (2,1) | {P, A, T, V} (3,1) |
| Configuration #2 | {P, A, T, V} (1,2) | - | {P, A, T, V} (3,2) |
| Configuration #3 | {P, A, T, V} (1,3) | {P, A, T, V} (2,3) | - |

A partir de ce tableau il est possible de choisir les configurations les plus efficaces, c'est-à-dire celles qui feront réagir le coeur de la façon la plus marquée suite au changement de la configuration d'origine à la configuration spécifique modifiée.

Ceci permettra de définir un "programme d'entraînement" consistant à appliquer de manière régulière, par exemple une fois par jour, une série de changements de configuration de stimulation pendant une durée précise et dans un ordre précis.

Dans un exemple de mise en oeuvre, ou peut ainsi prévoir d'appliquer des configurations spécifiques modifiées (configurations b) pendant 30 secondes à 1 minute, entrecoupées de périodes de récupération (retour à la configuration a de base) de 1 à 3 minutes.

Le choix du nombre de configurations modifiées, du nombre de changements de configurations ainsi que la durée de chaque configuration spécifique modifiée est paramétrable, notamment en fonction des résultats consignés dans le tableau ci-dessus qui permet de comparer l'efficacité des divers changements de configuration possibles.

Par exemple, les variations attendues des paramètres au changement de mode pourront être les suivantes :
- P(b,a) : excursion de ± 100 % par rapport à la valeur moyenne de PEA(i) dans la configuration a ;
- T(b,a) : environ 10 secondes ;
- A(b,a) : environ 50 % de la valeur moyenne de PEA(i) dans la configuration a ; et
- V(b,a) : environ 10% de A(b,a).

Ce programme d'entraînement permet de provoquer un remodelage inverse du coeur, bénéfique à long terme.

Chaque programme se compose de séquences, définies pour chacune par une configuration d'origine, une configuration spécifique temporaire, une durée d'application de cette configuration temporaire et une durée de récupération (retour à la configuration d'origine).

Il est possible de programmer plusieurs répétitions d'une même séquence, ou bien de programmer une succession de séquences différentes, avec une configuration spécifique modifiée à chaque nouvelle séquence.

La détermination des configurations les plus efficaces, sur le plan du remodelage inverse, est opérée en fonction de critères prédéterminés, la configuration spécifique sélectionnée (configuration b) étant par exemple celle présentant l'amplitude moyenne A(b,a) la plus basse pour l'amplitude maximale P(b,a) la plus élevée.

Ce choix peut se faire manuellement par le praticien sur visualisation du tableau indiqué plus haut, ou bien de façon automatique à partir d'une règle prédéterminée qui sera appliquée par l'implant.

On va décrire un exemple d'application d'un tel programme de séquences de changement de configuration.

Tout d'abord, le dispositif déclenche la première séquence programmée ; si la configuration courante est différente de la configuration d'origine a, une durée de récupération est appliquée, dans le mode a.

À la fin de cette durée de récupération éventuelle, l'appareil modifie la configuration, de a en b, pour une durée programmée.

Le signal EA est enregistré sur cette période, de manière à déterminer les données {P, A, T, V} caractérisant la transition de configuration. À la fin de la durée programmée, l'appareil revient à la configuration d'origine a, pendant une durée prescrite de récupération.

A la fin de cette récupération, le dispositif déclenche soit la séquence suivante, soit retourne à son mode de fonctionnement standard jusqu'à la prochaine phase d'entraînement (par exemple jusqu'au lendemain).

Le signal EA est enregistré sur l'ensemble des séquences et lorsque l'entraînement est terminé (fin de toutes les séquences), les données correspondantes sont stockées dans l'implant et/ou transmises à un programmateur extérieur.

Afin de fournir au praticien une information relative à l'impact du programme d'entraînement au fil des jours, plusieurs données sont sauvegardées et présentées, soit par transmission quotidienne, soit mémorisées dans l'implant pour être téléchargées lors de la prochaine consultation, au moyen du programmateur du praticien.

Ces données sont typiquement les valeurs {P, A, T, V} obtenues pour chaque séquence, complétées par l'amplitude PEA atteinte en fin de séquence, c'est-à-dire après application de la période de récupération.

Ces valeurs peuvent être complétées par d'autres données enregistrées dans une configuration de rythme et d'activité précise, par exemple : mesure quotidienne, la nuit, capteur d'activité indiquant un repos du patient, et fréquence cardiaque comprise entre la fréquence de base et 110 % de la fréquence de base. Ces données permettront au praticien de suivre l'impact de l'entraînement sur le coeur et évaluer l'efficacité de chaque séquence d'entraînement programmée sur le remodelage inverse recherché.

## Revendications

1. Un dispositif médical implantable actif de type prothèse cardiaque de resynchronisation par stimulation biventriculaire, comprenant :
- des moyens de détection d'événements auriculaires et ventriculaires ;
- des moyens aptes à appliquer des impulsions à des sites de stimulation des ventricules droit et gauche ;
- des moyens de calcul d'un délai atrioventriculaire, DAV ;
- des moyens de calcul d'un délai interventriculaire, DVV ;
la combinaison des sites de stimulation, du DAV et du DVV définissant ensemble une configuration de stimulation,
- des moyens de recueil d'un signal d'accélération endocardiaque (EA) ;
- des moyens de mesure de l'amplitude crête-à-crête (PEA(i)) du premier pic d'accélération endocardiaque, PEA, à chaque cycle cardiaque (i) ; et
- des moyens de commutation, aptes à changer de façon contrôlée et temporaire la configuration courante, d'une configuration originelle (a) à une configuration modifiée (b),
**caractérisé en ce qu'**il comprend en outre :
- des moyens aptes à évaluer, dans la configuration modifiée, un ensemble de données comprenant :
· la valeur maximale (P(b,a)) d'amplitude du PEA atteinte sur une première période temporelle après le changement de configuration ;
· la valeur moyenne (A(b,a)) du PEA, calculée sur un nombre prédéterminé de cycles cardiaques commençant après une deuxième période temporelle, de stabilisation, après le changement de configuration ;
· la variabilité (V(b,a)) du PEA autour de ladite valeur moyenne de PEA ; et
· la durée (T(b,a)) d'une troisième période temporelle, de stabilisation, après le changement de configuration.

2. Le dispositif de la revendication 1, comprenant en outre :
- des moyens de séquencement, pour appliquer des séquences de stimulation selon des configurations différentes, avec alternance, pendant des périodes temporelles prédéterminées, entre ladite configuration originelle et une configuration modifiée choisie parmi une pluralité de configurations modifiées différentes possibles, la configuration modifiée étant changée à chaque alternance.

3. Le dispositif de la revendication 3, dans lequel les moyens de séquencement sont aptes, après chaque application d'une configuration modifiée et retour à la configuration originelle, à maintenir cette configuration originelle pendant une durée de récupération prédéterminée.

4. Le dispositif de la revendication 1, comprenant en outre :
- des moyens aptes à constituer une table à deux entrées donnant ledit ensemble de données évalué pour chaque couple {configuration originelle, configuration modifiée} successivement appliqué.

5. Le dispositif de la revendication 1, comprenant en outre :
- des moyens pour désigner une configuration représentative de la réaction maximale du myocarde au changement de configuration.

6. Le dispositif de la revendication 5, dans lequel les moyens pour désigner une configuration représentative sont des moyens aptes à désigner la configuration minimisant la valeur moyenne du PEA par rapport à la valeur maximale d'amplitude du PEA.

7. Le dispositif de la revendication 1, comprenant des moyens pour évaluer ladite durée de troisième période temporelle, de stabilisation, après le changement de configuration par recherche de la durée nécessaire pour qu'une moyenne à court terme du PEA soit égale à une moyenne à long terme du PEA, à un écart autorisé prédéterminé près.

8. Le dispositif de la revendication 1, comprenant des moyens pour évaluer la variabilité du PEA autour de ladite valeur moyenne de PEA par calcul d'un écart-type du PEA sur un nombre prédéterminé de cycles consécutifs.

## Patentansprüche

1. Aktive implantierbare medizinische Vorrichtung nach Art einer Herzprothese zur Resynchronisation durch biventrikuläre Stimulation, umfassend:
- Mittel zum Erkennen von atrialen und ventrikulären Ereignissen;
- Mittel, die geeignet sind, um Impulse an Stimulationsorten der rechten und linken Herzkammern anzulegen;
- Mittel zum Berechnen einer atrioventrikulären Verzögerung, DAV;
- Mittel zum Berechnen einer interventrikulären
Verzögerung, DVV;
wobei die Kombination der Stimulationsorte der DAV und der DVV zusammen eine Stimulationskonfiguration definieren,
- Mittel zum Sammeln eines endokardialen Beschleunigungssignals (EA);
- Mittel zum Messen der Doppelamplitude (PEA(i)) der ersten endokardialen Beschleunigungsspitze, PEA, in jedem Herzzyklus (i); und
- Schaltmittel, die geeignet sind, um auf geregelte und vorübergehende Art und Weise die aktuelle Konfiguration von einer ursprünglichen Konfiguration (a) in eine geänderte Konfiguration (b) zu ändern,
**dadurch gekennzeichnet, dass** sie ferner Folgendes umfasst:
- Mittel die geeignet sind, um in der geänderten Konfiguration eine Datenmenge auszuwerten, die Folgendes umfasst:
· den maximalen Amplitudenwert (P(b,a)) der PEA, der in einem ersten Zeitraum nach der Konfigurationsänderung erreicht wird;
· den Mittelwert (A(b,a)) der PEA, der über eine vorbestimmte Anzahl von Herzzyklen berechnet wird, die nach einem zweiten Stabilisierungszeitraum nach der Konfigurationsänderung beginnen;
· die Variabilität (V(b,a)) der PEA um den PEA-Mittelwert; und
· die Dauer (T(b,a)) eines dritten Stabilisierungszeitraums nach der Konfigurationsänderung.

2. Vorrichtung nach Anspruch 1, ferner umfassend:
- Sequenzierungsmittel, um abwechselnd Stimulationssequenzen gemäß unterschiedlichen Konfigurationen während vorbestimmten Zeiträumen zwischen der ursprünglichen Konfiguration und einer geänderten Konfiguration, die aus einer Vielzahl möglicher unterschiedlicher geänderter Konfigurationen gewählt wird, anzuwenden, wobei die geänderte Konfiguration bei jedem Wechsel geändert wird.

3. Vorrichtung nach Anspruch 2, wobei die Sequenzierungsmittel geeignet sind, um nach jeder Anwendung einer geänderten Konfiguration und Rückkehr zur ursprünglichen Konfiguration, diese ursprüngliche Konfiguration während einer vorbestimmten Wiederherstellungsdauer beizubehalten.

4. Vorrichtung nach Anspruch 1, ferner umfassend:
- Mittel, die geeignet sind, um eine Tabelle mit zwei Eingaben zu bilden, welche die Datenmenge ergibt, die für jedes nacheinander angewendete Paar {ursprüngliche Konfiguration, geänderte Konfiguration} bewertet wird.

5. Vorrichtung nach Anspruch 1, ferner umfassend:
- Mittel zum Bezeichnen einer repräsentativen Konfiguration der maximalen Reaktion des Herzmuskels auf die Konfigurationsänderung.

6. Vorrichtung nach Anspruch 5, wobei die Mittel zum Bezeichnen einer repräsentativen Konfiguration Mittel sind, die geeignet sind, um die Konfiguration zu bezeichnen, die den Mittelwert der PEA im Verhältnis zum maximalen Amplitudenwert der PEA minimiert.

7. Vorrichtung nach Anspruch 1, umfassend Mittel zum Bewerten der Dauer des dritten Stabilisierungszeitraums nach der Konfigurationsänderung durch Suchen der Dauer, die notwendig ist, damit ein kurzfristiger Mittelwert der PEA gleich einem langfristigen Mittelwert der PEA ist, bis auf eine vorbestimmte zulässige Abweichung.

8. Vorrichtung nach Anspruch 1, umfassend Mittel zum Bewerten der Variabilität der PEA um den PEA-Mittelwert durch Berechnung einer Standardabweichung der PEA über eine vorbestimmte Anzahl aufeinanderfolgender Zyklen.

## Claims

1. An active implantable medical device such as a cardiac prosthesis for resynchronization by biventricular stimulation, comprising:
- means for detecting atrial and ventricular events;
- means adapted to apply pulses to stimulation sites of the right and left ventricles;
- means for calculating an atrioventricular delay, AVD;
- means for calculating an interventricular delay, VVD;
the combination of the stimulations sites, the AVD and the VVD defining together a stimulation configuration,
- means for collecting an endocardiac acceleration signal (EA);
- means for measuring the peak-to-peak amplitude (PEA(i)) of the first peak of endocardiac acceleration, PEA, in each cardiac cycle (i); and
- switching means, adapted to change in a controlled and temporary manner the current configuration, from an original configuration (a) to a modified configuration (b),
**characterized in that** it further comprises:
- means adapted to evaluate, in the modified configuration, a set of data comprising:
· the maximum value (P(b,a)) of amplitude of the PEA reached over a first time period after the change of configuration;
· the mean value (A(b,a)) of the PEA, calculated over a predetermined number of cardiac cycles beginning after a second time period, of stabilisation, after the change of configuration;
· the variability (V(b,a)) of the PEA about said mean value of PEA; and
· the duration (T(b,a)) of a third time period, of stabilisation, after the change of configuration.

2. The device of claim 1, further comprising:
- sequencing means, for applying sequences of stimulation according to different configurations, with alternation, during predetermined time periods, between said original configuration and a modified configuration chosen among a plurality of different possible modified configurations, the modified configuration being changed at each alternation.

3. The device of claim 2, wherein the sequencing means are adapted, after each application of a modified configuration and return to the original configuration, to hold this original configuration during a predetermined recovery time.

4. The device of claim 1, further comprising:
- means adapted to make a two-entry tables providing said data set evaluated for each couple {original configuration, modified configuration} successively applied.

5. The device of claim 1, further comprising:
- means for designating a configuration representative of the maximum reaction of the myocardium to the change of configuration.

6. The device of claim 5, wherein the means for designating a representative configuration are means adapted to designate the configuration minimizing the mean value of the PEA with respect to the maximum value of amplitude of the PEA.

7. The device of claim 1, comprising means for evaluating said duration of third time period, of stabilisation, after the change of configuration, by searching for the time necessary so that a short-term mean value of the PEA is equal to a long-term mean value of the PEA, within a predetermined allowed deviation.

8. The device of claim 1, comprising means for evaluating the variability of the PEA about said mean value of PEA, by calculating a standard deviation of the PEA over a predetermined number of consecutive cycles.
